# EUROPÄISCHE PATENTSCHRIFT

(11) **EP 4 281 124 B1**
(45) Veröffentlichungstag und Bekanntmachung des Hinweises auf die Patenterteilung: **14.10.2026**
(21) Anmeldenummer: 21843904.0
(22) Anmeldetag: 20.12.2021
(51) Int. Cl.: A61L 2/20, A61L 9/015, B01D 53/00, B01L 1/00, F24F 3/167

(54) **EINE PHARMAZEUTISCHE ANLAGE UND EIN VERFAHREN ZUM BETREIBEN EINER PHARMAZEUTISCHEN ANLAGE**
PHARMACEUTICAL UNIT AND METHOD FOR OPERATING A PHARMACEUTICAL UNIT
UNITÉ PHARMACEUTIQUE ET PROCÉDÉ DE FONCTIONNEMENT D'UNE UNITÉ PHARMACEUTIQUE

(30) Priorität: 22.01.2021 DE 102021101403
(43) Veröffentlichungstag der Anmeldung: 29.11.2023
(73) Patentinhaber: Syntegon Technology GmbH, 70372 Stuttgart (DE)
(72) Erfinder: KÜHNLE, Albrecht, 74564 Crailsheim (DE); HOFFMANN, Benjamin, 74541 Vellberg (DE); KOSIAN, Thomas, 91610 Insingen (DE)
(74) Vertreter: Novagraaf Group
(86) Internationale Anmeldenummer: PCT/EP2021/086810
(87) Internationale Veröffentlichungsnummer: WO 2022/156976

(56) Entgegenhaltungen:
- US-A1- 2005 163 685
- US-A1- 2013 336 844

## Beschreibung

Die Erfindung betrifft eine pharmazeutische Anlage, insbesondere einen Reinraum, mit den Merkmalen des Oberbegriffs des Anspruchs 1 und ein Verfahren zum Betreiben einer pharmazeutischen Anlage mit Merkmalen des nebengeordneten Anspruchs.

Pharmazeutische Produktionsanlagen sind in der Regel in Form von Barrieresystemen ausgebildet, die gegenüber ihrer Umgebung abgeschlossen sind.

Bei pharmazeutischen Produktionseinrichtungen mit hoher Staub und/oder Aerosolbelastung, insbesondere, wenn diese Belastungen zudem als hoch potent eingestuft werden, werden sogenannte BIBO- (bag-in/bag-out) Filtersysteme in der Um- bzw. Abluft der Produktionsbereiche eingesetzt. Diese sind speziell für einen kontaminationsarmen Wechsel der beladenen Filterelemente bei Erreichen der maximalen Filterbelastung oder am Ende eines definierten Wechselintervalls ausgebildet.

Alternativ werden auch abreinigbare Filtersysteme eingesetzt, bei denen kein Filterwechsel erforderlich ist. Diese lassen jedoch keine hinreichend vollständige Abreinigung des Filtermediums zu.

Pharmazeutische Anlagen müssen regelmäßig dekontaminiert werden. Dazu wird Dekontaminationsmittel in die pharmazeutische Anlage bzw. das Barrieresystem eingeleitet, so dass alle kontaminierten Elemente in Kontakt mit dem Dekontaminationsmittel gelangen und so dekontaminiert werden.

Wird als Dekontaminationsmittel ein Mittel eingesetzt, dass unschädlich für die Umwelt ist, spricht man von einer Biodekontamination. Üblicherweise wird hierzu Wasserstoffperoxid (H2O2) eingesetzt, da dieses zu Wasser und Sauerstoff abgebaut werden kann.

Untersuchungen haben gezeigt, dass Beladung der Filtersysteme Auswirkungen auf die Wirksamkeit der (Bio-)Dekontamination eines Barrieresystems haben kann. Insbesondere bei Barrieresystemen, die in pulververarbeitenden Produktionsanlagen verwendet werden, treten in der Umluft hohe Staubkonzentrationen mit entsprechend hoher Beladung der Umluftfilter auf. Mit Umluft ist Luft aus dem Barrieresystem, die rezirkuliert wird, gemeint.

In der Regel handelt es sich zudem um hygroskopische Stäube, die bewirken, dass während der (Bio-)Dekontamination ein Großteil des gasförmigen Dekontaminationsmittels der Umluft in diesen Stäuben gebunden werden. Handelt es sich dabei um ein in Rezirkulation betriebenes Barrieresystem, so steht diese in Stäuben gebundene Konzentration des Dekontaminationsmittels nicht mehr für die Dekontamination des Gesamtsystems zur Verfügung.

In der Praxis werden die Validierung und Qualifizierung von solchen Anlagen mit neuen (unbeladenen) Filtern durchgeführt, sodass dieser Effekt erst im Betrieb nach mehreren Betriebsstunden eintritt. Es besteht somit die Gefahr, dass solche Anlagen, quasi unbemerkt, nicht konform betrieben werden.

US 2005 / 163 685 A1 und US 2013 / 336 844 A1 offenbaren jeweils eine Anlage mit Merkmalen des Anspruchs 1.

Die Aufgabe der vorliegenden Erfindung besteht darin, eine pharmazeutische Anlage und ein Verfahren zum Betreiben einer pharmazeutischen Anlage bereitzustellen, wobei die obigen Nachteile ausgeräumt werden.

Diese Aufgabe wird durch eine pharmazeutische Anlage, insbesondere einen Reinraum, mit den Merkmalen des Anspruchs 1 gelöst, wobei die pharmazeutische Anlage umfasst:
Einen gegenüber seiner Umgebung abgeschlossenen Arbeitsraum und ein Filtersystem mit einem ersten Strömungspfad und einem ersten Filter.

Der erste Filter ist dabei in dem ersten Strömungspfad angeordnet. Der erste Filter ist ausgebildet, um Luft aus dem Arbeitsraum, zu filtern. Damit wird die Luft aus dem Arbeitsraum, welche über den ersten Strömungspfad geleitet wird, mittels des ersten Filters gefiltert.

Das Filtersystem umfasst einen zweiten Strömungspfad und eine Umschalteinrichtung. Die Umschalteinrichtung ist ausgebildet, um während einer Betriebsphase der pharmazeutischen Anlage Luft aus dem Arbeitsraum, über den ersten Strömungspfad und somit über den ersten Filter zu leiten.

Die Umschalteinrichtung ist weiter ausgebildet, um während einer Dekontaminationsphase der pharmazeutischen Anlage Luft aus dem Arbeitsraum, ausschließlich über den zweiten Strömungspfad zu leiten. Damit wird der erste Filter während der Dekontaminationsphase nicht mit Dekontaminationsmittel beaufschlagt. Der erste Filter wird während der Dokumentationsphase nicht mit Luft aus dem Arbeitsraum beaufschlagt.

Der erste und der zweite Strömungspfad können sich in zumindest einem Bereich überschneiden. Der erste und der zweite Strömungspfad können innerhalb zumindest eines Bereiches gemeinsam, beispielsweise innerhalb derselben Leitung, verlaufen. Der erste und der zweite Strömungspfad können zumindest einem Abschnitt miteinander fluidisch gekoppelt sein. Verlaufen der erste und der zweite Strömungspfad gemeinsam, bzw. überschneiden sich in einem Abschnitt, so liegt jedoch der erste Filter außerhalb des gemeinsamen Abschnitts und wird nur dann mit Luft aus dem Arbeitsraum beaufschlagt, wenn Luft über den ersten Strömungspfad geleitet wird.

Vorliegend ist mit "ausschließlich über den ersten Strömungspfad geleitet" gemeint, dass nur der erste Strömungspfad komplett entlang seiner gesamten Erstreckung) mit Luft aus dem Arbeitsraum durchströmt wird. Dabei können beispielsweise auch gemeinsame Abschnitte des ersten Strömungspfades und des zweiten Strömungspfades mit Luft durchströmt werden. Jedenfalls wird der zweite Strömungspfad nicht komplett (entlang seiner gesamten Erstreckung) mit Luft aus dem Arbeitsraum durchströmt, wenn von einer ausschließlichen Durchströmung des ersten Strömungspfades die Rede ist, sondern allenfalls entlang seiner mit dem ersten Strömungspfad ggf. gemeinsamen Abschnitte.

Analog ist mit "ausschließlich über den zweiten Strömungspfad geleitet" gemeint, dass nur der zweite Strömungspfad komplett (entlang seiner gesamten Erstreckung) mit Luft aus dem Arbeitsraum durchströmt wird. Dabei können beispielsweise auch gemeinsame Abschnitte des zweiten Strömungspfades und des ersten Strömungspfades mit Luft durchströmt werden. Jedenfalls wird der erste Strömungspfad nicht komplett (entlang seiner gesamten Erstreckung) mit Luft aus dem Arbeitsraum durchströmt, wenn von einer ausschließlichen Durchströmung des zweiten Strömungspfades die Rede ist, sondern allenfalls entlang seiner mit dem zweiten Strömungspfad ggf. gemeinsamen Abschnitte.

Vorliegend ist mit einer Leitung von Luft über den ersten Strömungspfad bzw. einer Durchströmung des ersten Strömungspfades gemeint, dass die Luft den ersten Strömungspfad komplett also entlang seiner gesamten Erstreckung durchströmt.

Analog hierzu ist mit einer Leitung von Luft über den zweiten Strömungspfad bzw. einer Durchströmung des zweiten Strömungspfades gemeint, dass die Luft den zweiten Strömungspfad komplett durchströmt.

Die Dekontamination kann mittels Einleitung von Dekontaminationsmittel in den Arbeitsraum realisiert werden. Dabei kann das Dekontaminationsmittel gasförmig sein. Bei der Dekontamination kann es sich um Biodekontamination handeln. Insbesondere kann das Dekontaminationsmittel Wasserstoffperoxid (H2O2) sein. Ebenso kann das Dekontaminationsmittel ein Gemisch aus Wasserstoffperoxid und Luft sein. Denkbar ist auch, dass das Dekontaminationsmittel ein Gemisch aus Wasserstoffperoxid und mindestens einem Gas ist.

Auf diese Weise wird das Dekontaminationsmittel an dem ersten Filter umgeleitet. Damit gelangt kein Dekontaminationsmittel in bzw. durch den gegebenenfalls beladenen ersten Filter. Mit anderen Worten, der erste Filter wird umgangen. Mit anderen Worten, der erste Strömungspfad wird während der Dekontamination bzw. Dekontaminationsphase nicht mit Dekontaminationsmittel und/oder Luft durchströmt.

So wird, insbesondere bei aseptischen Pulveranwendungen, das Dekontaminationsmittel nicht durch den ersten (gegebenenfalls beladenen) Filter aus dem Dekontaminationsvorgang herausgefiltert und steht im vollen Umfang dem Dekontaminationsprozess während der Dekontaminationsphase zur Verfügung.

Mit Herausfiltern ist gemeint, dass das Dekontaminationsmittel durch den ersten Filter oder dessen Beladung (chemisch oder physikalisch) gebunden bzw. zurückgehalten wird und nicht mehr dem Dekontaminationsprozess zur Verfügung steht.

Bei der Luft handelt es sich um Umluft aus dem Arbeitsraum. Mit Umluft ist Luft gemeint, die in dem Arbeitsraum rezirkuliert wird. Mit Abluft ist Luft gemeint, die aus dem Arbeitsraum abgeleitet wird. Ebenso ist es denkbar, dass es sich bei der Luft um in den Arbeitsraum eingeleitete Außenluft und/oder aufbereitete (bspw. vorgefilterte) Außenluft handelt. Selbstverständlich kann anstelle von Luft auch um ein anderes Gas bzw. Gasgemisch benutzt werden.

Das Filtersystem umfasst einen zweiten Filter, der ausgebildet ist, um Luft aus dem Arbeitsraum, zu filtern. Dabei ist der zweite Filter in dem zweiten Strömungspfad angeordnet, sodass Luft aus dem Arbeitsraum, welche über den zweiten Strömungspfad geleitet wird, mittels des zweiten Filters gefiltert wird.

Damit kann der gegebenenfalls beladene erste Filter während der (Bio-) Dekontaminationsphase durch einen zweiten, unbelasteten Filter umgangen werden. Damit kann bewirkt werden, dass das (Bio-) Dekontaminationsmittel auch nach erfolgter Rezirkulation mit maximaler, reprodurzierbarer Wirksamkeit dem Dekontaminationsvorgang zur Verfügung steht.

Die Umschalteinrichtung kann mindestens ein Ventil umfassen. Das Ventil kann ausgebildet sein, um den ersten Strömungspfad und fluidisch mit dem Arbeitsraum zu koppeln oder zu entkoppeln. Alternativ oder zusätzlich kann das Ventil ausgebildet sein, um den zweiten Strömungspfad fluidisch mit dem Arbeitsraum zu koppeln oder zu entkoppeln. Insbesondere kann die Umschalteinrichtung zwei Ventile umfassen, wobei diese derart eingerichtet sein können, dass ein erstes Ventil den ersten Strömungspfad fluidisch mit dem Arbeitsraum koppeln oder entkoppeln kann. Das zweite Ventil kann dabei den zweiten Strömungspfad fluidisch mit dem Arbeitsraum koppeln oder entkoppeln.

Mit einer fluidischen Koppelung ist gemeint, dass ein Fluid (Gas oder Flüssigkeit) frei zwischen mindestens zwei fluidisch gekoppelten Elementen fließen kann. Entsprechend ist mit fluidischen Entkopplung gemeint, dass ein Fluid (Gas oder Flüssigkeit) nicht zwischen zwei fluidisch entkoppelten Elementen fließen kann.

Der erste Filter kann innerhalb der pharmazeutischen Anlage insbesondere innerhalb des Arbeitsraumes angeordnet sein. Alternativ oder zusätzlich kann der zweite Filter innerhalb der pharmazeutischen Anlage, insbesondere innerhalb des Arbeitsraumes, angeordnet sein. Insbesondere kann das gesamte Filtersystem mit allen Komponenten innerhalb der pharmazeutischen Anlage, insbesondere innerhalb des Arbeitsraumes, angeordnet sein.

Es ist aber auch denkbar, dass der erste Filter außerhalb des Arbeitsraumes, insbesondere außerhalb der pharmazeutischen Anlage, angeordnet sein kann. Alternativ oder zusätzlich kann der zweite Filter außerhalb des Arbeitsraumes, insbesondere außerhalb der pharmazeutischen Anlage, angeordnet sein. Insbesondere kann das gesamte Filtersystem mit allen Komponenten außerhalb des Arbeitsraumes, insbesondere außerhalb der pharmazeutischen Anlage, angeordnet sein. Dabei kann das Filtersystem oder einzelne Komponenten des Filtersystems, insbesondere mittels Leitungen, mit der pharmazeutischen Anlage, insbesondere mit dem Arbeitsraum fluidisch gekoppelt sein.

Das Filtersystem kann derart ausgebildet sein, dass während der Betriebsphase der pharmazeutischen Anlage Luft aus dem Arbeitsraum über den ersten Filter und den zweiten Filter geleitet wird. Damit kann der zweite Filter während der Betriebsphase die Funktion des ersten Filters übernehmen.

Der erste Filter und der zweite Filter sind fluidisch in Reihe zu einander angeordnet. Dabei ist der zweite Filter dem ersten Filter nachgeschaltet. Insbesondere kann der zweite Filter ein Polizeifilter sein. Damit kann der zweite Filter eine zusätzliche Filter- als auch eine Sicherungsfunktion übernehmen. Sollte der erste Filter ausfallen, kann der zweite Filter die Filterfunktion des ersten Filters übernehmen, so dass eine ordnungsgemäße Filterung aufrechterhalten werden kann. Dabei leitet der zweite Strömungspfad die Luft aus dem Arbeitsraum parallel um den ersten Filter und durch den zweiten Filter.

Der erste Filter und der zweite Filter können fluidisch parallel zueinander angeordnet sein. Somit kann die Luft über den ersten Strömungspfad (z. B. während der Betriebsphase) und somit über den ersten Filter oder über den zweiten Strömungspfad (z. B. während der Dekontaminationsphase) und somit über den zweiten Filter geleitet werden.

Die Umschalteinrichtung kann ausgebildet sein, um während der Betriebsphase der pharmazeutischen Anlage Luft aus dem Arbeitsraum, ausschließlich über den ersten Strömungspfad zu leiten. Mit anderen Worten, es wird keine Luft aus dem Arbeitsraum über den zweiten Strömungspfad und somit über den zweiten Filter während des Betriebsphase der pharmazeutischen Anlage geleitet. Auf diese Weise kann die Belastung (Verschmutzung) des zweiten Strömungspfads bzw. Des zweiten Filters verhindert oder zumindest minimiert werden.

Weiter wird die zu lösende Aufgabe durch ein Verfahren zum Betreiben einer pharmazeutischen Anlage mit einem gegenüber seiner Umgebung abgeschlossenem Arbeitsraum mit den Merkmalen des nebengeordneten Anspruchs gelöst, wobei die pharmazeutische Anlage ein Filtersystem umfasst mit einem ersten Strömungspfad und einem ersten Filter.

Dabei ist der erste Filter in dem ersten Strömungspfad angeordnet und ist ausgebildet, um Luft, die aus dem Arbeitsraum über den ersten Strömungspfad geleitet wird, zu filtern.

Das Filtersystem umfasst einen zweiten Strömungspfad und eine Umschalteinrichtung. Dabei kann mittels der Umschalteinrichtung die Strömung der Luft aus dem Arbeitsraum zwischen dem ersten Strömungspfad und dem zweiten Strömungspfad geschalten werden.

Das Verfahren umfasst:
Leiten von Luft aus dem Arbeitsraum über den ersten Strömungspfad in einer Betriebsphase der pharmazeutischen Anlage und leiten von Luft aus dem Arbeitsraum ausschließlich über den zweiten Strömungspfad, insbesondere über den zweiten Filter, während einer Dekontaminationsphase der pharmazeutischen Anlage.

Die Dekontamination kann mittels Einleitung von Dekontaminationsmittel in den Arbeitsraum realisiert werden. Dabei kann das Dekontaminationsmittel gasförmig sein. Bei der Dekontamination kann es sich um Biodekontamination handeln. Insbesondere kann das Dekontaminationsmittel Wasserstoffperoxid (H2O2) sein. Ebenso kann das Dekontaminationsmittel ein Gemisch aus Wasserstoffperoxid und Luft sein. Denkbar ist auch, dass das Dekontaminationsmittel ein Gemisch aus Wasserstoffperoxid und mindestens einem Gas ist.

Zum Betreiben der pharmazeutischen Anlage gehört sowohl Betriebsphase der pharmazeutischen Anlage als auch die Dekontaminationsphase der pharmazeutischen Anlage.

Während der Betriebsphase der pharmazeutischen Anlage kann die Luft aus dem Arbeitsraum, ausschließlich über den ersten Strömungspfad und damit den ersten Filter geleitet werden und somit ausschließlich mittels des ersten Filters gefiltert werden.

Während der Betriebsphase der pharmazeutischen Anlage kann die Luft aus dem Arbeitsraum über den ersten Strömungspfad und den ersten Filter geleitet werden und zusätzlich über den zweiten Strömungspfad und über den zweiten Filter geleitet werden. Somit kann die Luft aus dem Arbeitsraum während der Betriebsphase der pharmazeutischen Anlage mittels des ersten Filters und zusätzlich mittels des zweiten Filters gefiltert werden. Dabei ist der Zweite Filter dem ersten Filter in Strömungsrichtung nachfolgend angeordnet. Der zweite Filter wird entsprechend nur mit bereits durch den ersten Filter gefilterter Luft beaufschlagt, wenn die Luft über den ersten Strömungsweg geleitet wird.

Zur Durchführung des Verfahrens wird eine pharmazeutische Anlage , insbesondere ein Reinraum, mit oben beschriebenen Merkmalen benutzt.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine schematische Darstellung einer pharmazeutischen Anlage während einer Betriebsphase;
- Fig. 2: eine schematische Darstellung der pharmazeutischen Anlage gem. Fig. 1 während einer Dekontaminationsphase;
- Fig. 3: eine schematische Darstellung eines weiteren Ausführungsbeispiels der pharmazeutischen Anlage;
- Fig. 4: eine schematische Darstellung der pharmazeutischen Anlage gem. Fig. 3 während der Betriebsphase;
- Fig. 5: eine schematische Darstellung der pharmazeutischen Anlage gem. Fig. 3 während der Dekontaminationsphase und
- Fig. 6: eine schematische Darstellung eines weiteren Ausführungsbeispiels der pharmazeutischen Anlage.

In der nachfolgenden Beschreibung sowie in den Figuren tragen sich entsprechende Bauteile und Elemente gleiche Bezugszeichen. Der besseren Übersichtlichkeit wegen sind nicht in allen Figuren sämtliche Bezugszeichen wiedergegeben.

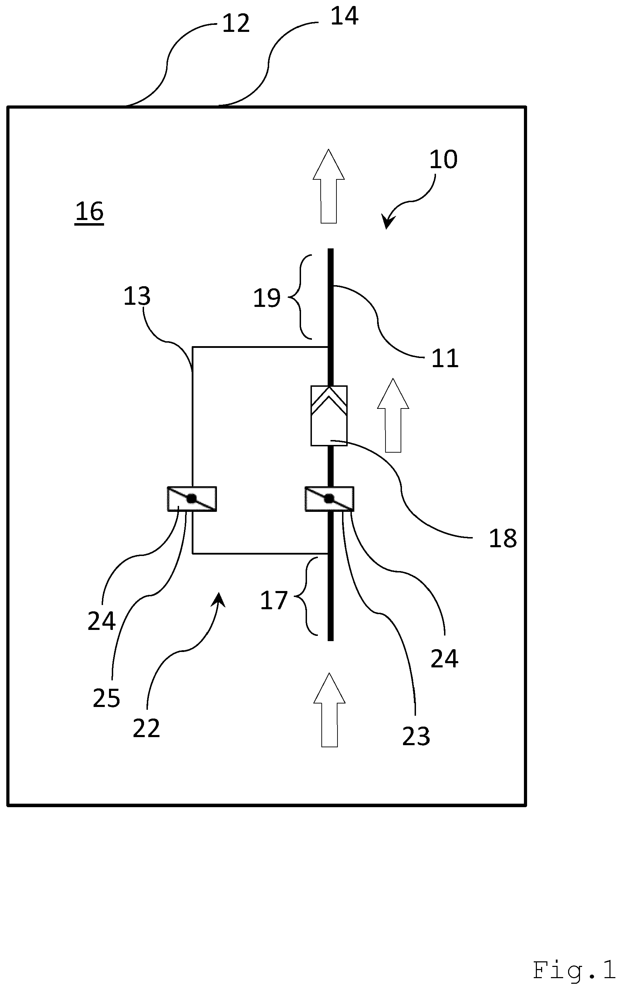

Figur 1 zeigt eine schematische Darstellung einer pharmazeutischen Anlage 12 während einer Betriebsphase. Vorliegend ist die pharmazeutische Anlage 12 in Form eines Reinraums 14 ausgebildet. Der Reinraum 14 weist einen Arbeitsraum 16 auf. Dieser ist gegenüber seiner Umgebung abgeschlossen.

Der Reinraum 14 weist weiter ein Filtersystem 10 auf. Vorliegend ist das Filtersystem 10 innerhalb des Arbeitsraums 16 angeordnet. Dass das Filtersystem 10 weist einen ersten Strömungspfad 11 und einen ersten Filter 18 auf.

Das Filtersystem 10 weist zudem einen zweiten Strömungspfad 13 und eine Umschalteinrichtung 22 auf. Die Umschalteinrichtung 22 ist derart ausgebildet, dass Luft aus dem Arbeitsraum 16, wahlweise über den ersten Strömungspfad 11 und somit über den ersten Filter 18 oder über den zweiten Strömungspfad 13 geleitet werden kann. Hierzu umfasst die Umschalteinrichtung 22 zwei Ventile 24. In dem dargestellten Ausführungsbeispiel ist ein erstes Ventil 23 innerhalb des ersten Strömungspfades 11 und ein zweites Ventil 25 innerhalb des zweiten Strömungspfades 13 angeordnet.

Durch Öffnen/Schließen des ersten Ventils 23 kann der erste Strömungspfad 11 geöffnet bzw. geschlossen werden. Mit anderen Worten, mittels des ersten Ventils 23 kann der erste Strömungspfad 11 fluidisch mit dem Arbeitsraum 16 gekoppelt bzw. entkoppelt werden.

Entsprechend kann der zweite Strömungspfad 13 durch Öffnen/Schließen des zweiten Ventils 25 geöffnet bzw. geschlossen werden. Mit anderen Worten, mittels des zweiten Ventils 25 kann der zweite Strömungspfad 13 fluidisch mit dem Arbeitsraum 16 gekoppelt bzw. entkoppelt werden.

Figur 1 zeigt den Reinraum 14 in der Betriebsphase. Dabei wird Luft aus dem Arbeitsraum 16 in das Filtersystem 10 angesaugt und über den ersten Strömungspfad 11 und somit den ersten Filter 18 geleitet. In Figur 1 ist der erste Strömungspfad 11 dick hervorgehoben und der Weg der Luft, über den ersten Strömungspfad 11 mit drei dicken Pfeilen angedeutet.

In dem dargestellten Zustand (Betriebsphase) ist das erste Ventil 23 geöffnet, sodass die Luft aus dem Arbeitsraum 16 über den ersten Strömungspfad 11 und somit über den ersten Filter 18 fließen kann. Mit anderen Worten, der erste Strömungspfad 11 ist mittels des geöffneten ersten Ventils 23 fluidisch mit dem Arbeitsraum 16 gekoppelt.

In dem dargestellten Zustand (Betriebsphase) ist das zweite Ventil 25 geschlossen, sodass die Luft aus dem Arbeitsraum 16 nicht über den zweiten Strömungspfad 13 fließen kann. Mit anderen Worten, der zweite Strömungspfad 13 ist mittels des geschlossenen zweiten Ventils 25 fluidisch von dem Arbeitsraum 16 entkoppelt.

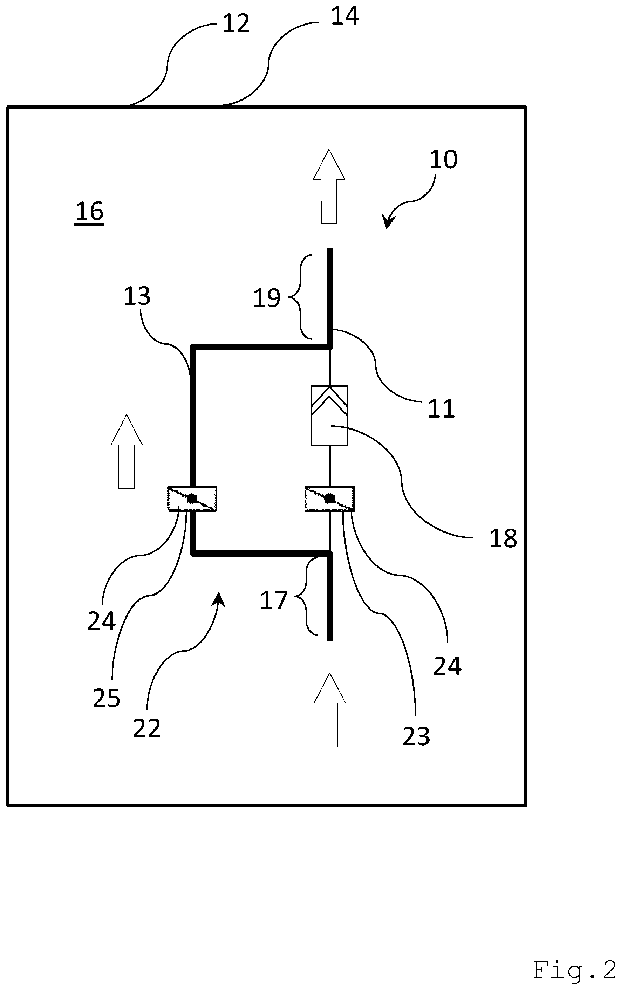

Figur 2 zeigt eine schematische Darstellung der pharmazeutischen Anlage gemäß Figur 1 während einer Dekontaminationsphase. Vorliegend ist der zweite Strömungspfad 13 mittels einer dicken Linie hervorgehoben.

Während der Dekontaminationsphase wird die pharmazeutische Anlage, insbesondere der Arbeitsraum 16, dekontaminiert.

Während der Dekontaminationsphase wird ein Dekontaminationsmittel (insbesondere gasförmiges Wasserstoffperoxid) in den Arbeitsraum 16 eingeleitet.

Alternativ oder zusätzlich kann das Dekontaminationsmittel in das Filtersystem 10, insbesondere in den zweiten Strömungspfad 13, eingeleitet werden.

Die Luft aus dem Arbeitsraum 16 und insbesondere das Dekontaminationsmittel werden in das Filtersystem 10 angesaugt und über den zweiten Strömungspfad 13 geleitet. Der Weg der Luft bzw. des Dekontaminationsmittels über den zweiten Strömungspfad 13 ist in Figur 2 mit drei dicken Pfeilen angedeutet.

In dem dargestellten Zustand (Dekontaminationsphase) ist das zweite Ventil 25 geöffnet, sodass die Luft aus dem Arbeitsraum 16 über den zweiten Strömungspfad 13 fließen kann. Mit anderen Worten, der zweite Strömungspfad 13 ist mittels des geöffneten zweiten Ventils 25 fluidisch mit dem Arbeitsraum 16 gekoppelt.

In dem dargestellten Zustand (Dekontaminationsphase) ist das erste Ventil 23 geschlossen, sodass die Luft aus dem Arbeitsraum 16 nicht über den ersten Strömungspfad 11 und somit über den ersten Filter 18 fließen kann. Mit anderen Worten, der erste Strömungspfad 11 und somit der erste Filter 18 sind mittels des geschlossenen ersten Ventils 23 fluidisch von dem Arbeitsraum 16 entkoppelt.

Mit anderen Worten, während der Dekontaminationsphase wird der erste Filter 18 nicht mit Luft bzw.

Dekontaminationsmittel durchströmt. Die Beladung des ersten Filters 18 bzw. die im Filter 18 während der Betriebsphase herausgefilterten Partikel können so nicht mit der Luft bzw. dem Dekontaminationsmittel wechselwirken. Das Dekontaminationsmittel steht so im vollen Umfang für den Dekontaminationsprozess während der Dekontaminationsphase zur Verfügung.

In den in Figuren 1 bis 6 gezeigten Ausführungsbeispielen weist der erste Strömungspfad 11 und der zweite Strömungspfad 13 erfindungsgemäß einen ersten gemeinsamen Abschnitt 17 auf und einen zweiten gemeinsamen Abschnitt 19 auf. Vorliegend verlaufend der erste Strömungspfad 11 und der zweite Strömungspfad 13 in den beiden gemeinsamen Abschnitten 17, 19 entlang einer gemeinsamen Leitung. Mit anderen Worten, der erste Strömungspfad 11 und der zweite Strömungspfad 13 sind entlang der gemeinsamen Abschnitten 17, 19 fluidisch gekoppelt.

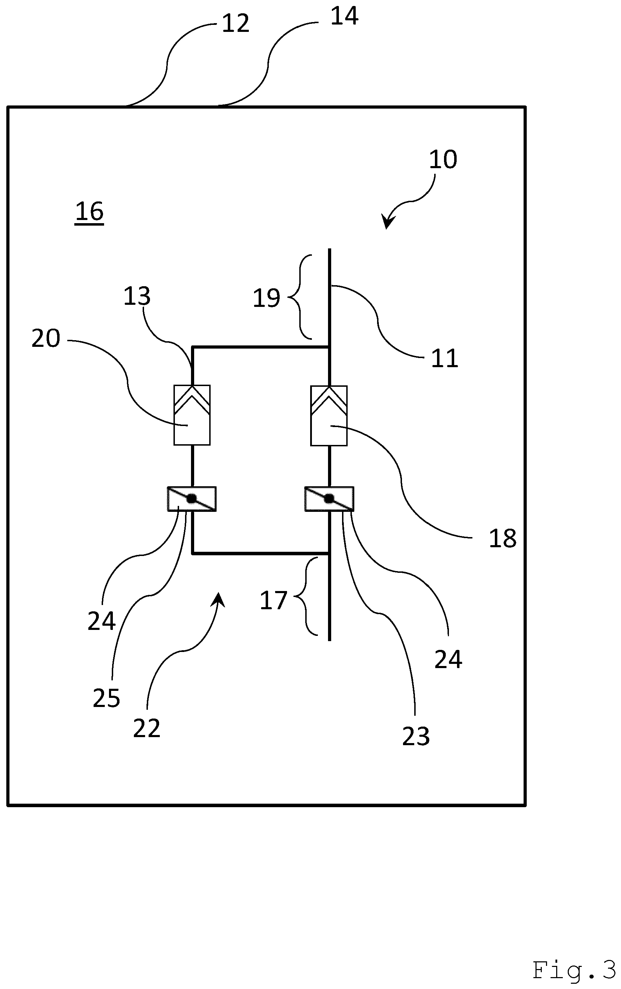

Figur 3 zeigt eine schematische Darstellung eines weiteren Ausführungsbeispiels der pharmazeutischen Anlage 12. Das dargestellte Ausführungsbeispiel unterscheidet sich von dem voranstehenden dadurch, dass ein zweiter Filter 20 innerhalb des zweiten Strömungspfades 13 angeordnet ist.

Wird die Luft aus dem Arbeitsraum 16 über den zweiten Strömungspfad 13 geleitet, so durchströmt die Luft auch den zweiten Filter 20.

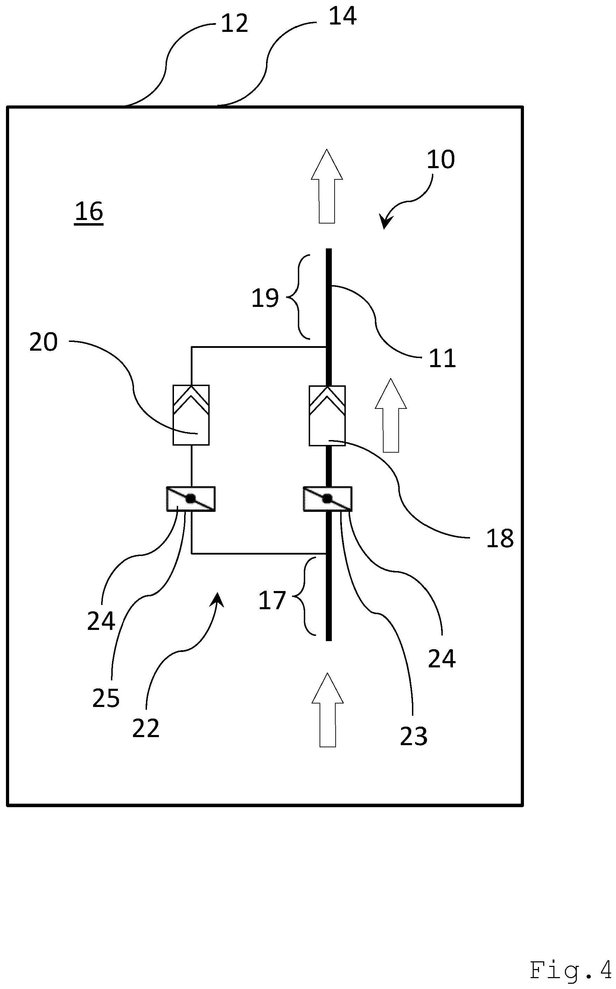

Figur 4 zeigt eine schematische Darstellung der pharmazeutischen Anlage 12 gemäß Figur 3 während der Betriebsphase.

Während der Betriebsphase wird die Luft aus dem Arbeitsraum 16 in das Filtersystem 10 angesaugt und über den ersten Strömungspfad 11 und somit über den ersten Filter 18 geleitet. Hierzu ist der erste Strömungspfad 11 mittels des geöffneten ersten Ventils 23 mit dem Arbeitsraum 16 fluidisch gekoppelt.

Vorliegend wird während der Betriebsphase der zweite Strömungspfad 13 und somit der zweite Filter 20 mittels eines geschlossenen zweiten Ventils 25 von dem Arbeitsraum 16 fluidisch entkoppelt. Mit anderen Worten, während der Betriebsphase strömt keine Luft über den zweiten Strömungspfad 13 bzw. den zweiten Filter 20. So kann verhindert werden, dass der zweite Strömungspfad 13 und insbesondere der zweite Filter 20 während der Betriebsphase beispielsweise durch während der Betriebsphase erzeugten Partikel beladen wird.

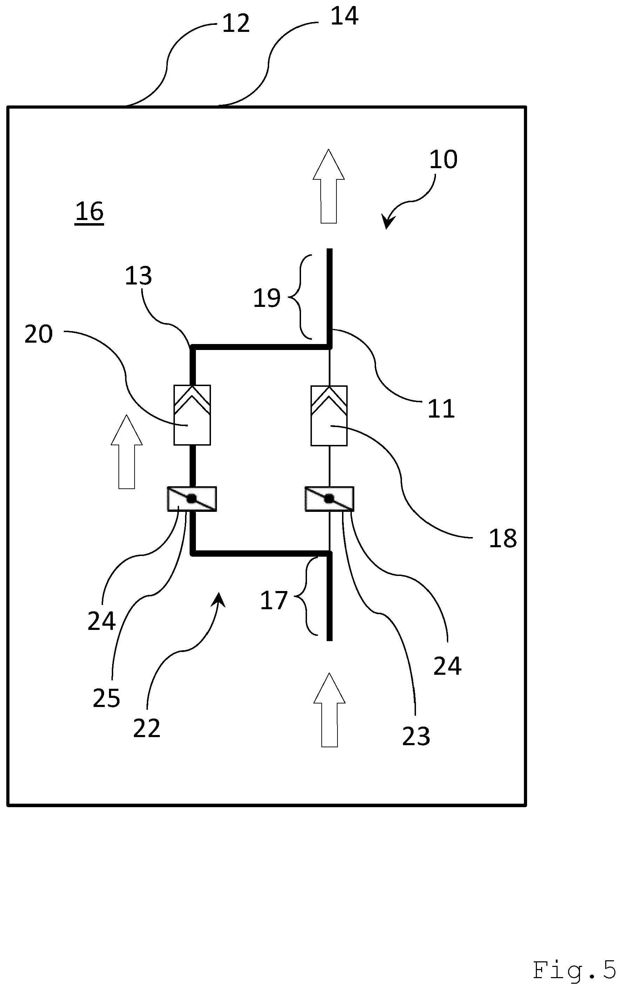

Figur 5 zeigt eine schematische Darstellung der pharmazeutischen Anlage 12 gemäß Figur 3 während der Dekontaminationsphase.

Während der Dekontaminationsphase bzw. der Dekontamination wird die Luft aus dem Arbeitsraum 16 in das Filtersystem 10 angesaugt und über den zweiten Strömungspfad 13 und somit über den zweiten Filter 20 geleitet. Der Weg der Luft durch den zweiten Strömungspfad 13 ist in Figur 5 mittels drei dicker Pfeile angedeutet.

Während der Dekontaminationsphase ist der erste Strömungspfad 11 mittels des geschlossenen ersten Ventils 23 von dem Arbeitsraum 16 fluidisch entkoppelt.

Während der Dekontaminationsphase ist der zweite Strömungspfad 13 und somit auch der zweite Filter 20 mittels des geöffneten zweiten Ventils 25 fluidisch mit dem Arbeitsraum 16 gekoppelt.

Somit wird das Dekontaminationsmittel nur über den zweiten Strömungspfad 13 und somit nur über den zweiten Filter 20 geleitet. Das Dekontaminationsmittel wird nicht über den ersten Strömungspfad 11 geleitet und gelangt somit nicht in den ersten Filter 18. Das Dekontaminationsmittel kann so nicht mit der Beladung des ersten Filters 18 wechselwirken, sodass das Dekontaminationsmittel nicht herausgefiltert werden kann (siehe oben) und in vollem Umfang für den Dekontaminationsprozess während der Dekontaminationsphase zur Verfügung steht.

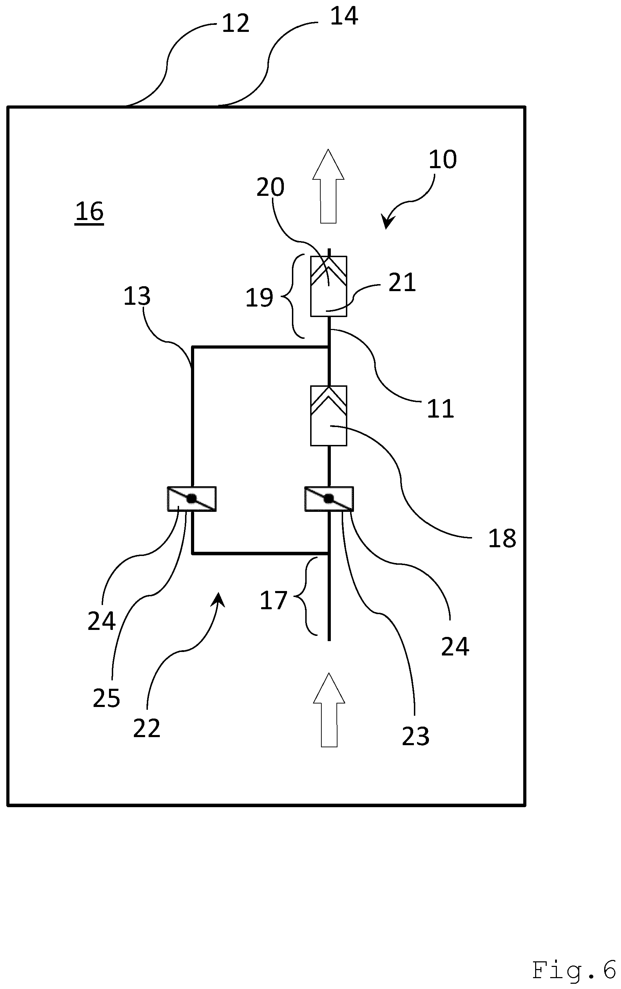

Figur 6 zeigt eine schematische Darstellung eines weiteren Ausführungsbeispiels der pharmazeutischen Anlage 12. Vorliegend ist der erste Filter 18 und der zweite Filter 21 fluidisch in Reihe geschaltet.

Erfindungsgemäß ist der erste Filter 18 innerhalb des ersten Strömungspfades 11 angeordnet. Erfindungsgemäß ist der zweite Filter 21 ist dem ersten Filter 18 nachgeschaltet und innerhalb des gemeinsamen Abschnitts 19 des ersten Strömungspfades 11 und des zweiten Strömungspfades 13 angeordnet.

In der dargestellten Anordnung übernimmt der zweite Filter 20 die Funktion eines sogenannten Polizeifilters 21. in der Betriebsphase wird die Luft aus dem Arbeitsraum 16 in das Filtersystem 10 angesaugt und über den ersten Strömungspfad 11 geleitet.

Hierzu ist das erste Ventil 23 geöffnet, sodass der erste Strömungspfad 11 fluidisch mit dem Arbeitsraum 16 gekoppelt ist. Das zweite Ventil 25 ist geschlossen, sodass der zweite Strömungspfad 13 fluidisch vom Arbeitsraum 16 entkoppelt ist.

Die Luft, welche durch den ersten Strömungspfad 11 geleitet wird, wird mittels des ersten Filters 18 gefiltert. Die mittels des ersten Filters 18 gefilterte Luft wird weiter über den gemeinsamen Abschnitt 19 und über den zweiten Filter 20 geleitet. Der zweite Filter 20 übernimmt eine Sicherungsfunktion. Sollte der erste Filter 18 während der Betriebsphase ausfallen, so kann der zweite Filter 20 (als Polizeifilter 21) die Funktion des ersten Filters 18 übernehmen und somit die Filterfunktion des Filtersystems 10 gewährleisten.

Für die Dekontaminationsphase wird das erste Ventil 23 geschlossen und das zweite Ventil 25 geöffnet. Damit wird die aus dem Arbeitsraum 16 in das Filtersystem 10 angesaugte Luft bzw. das Dekontaminationsmittel über den zweiten Strömungspfad 13 geleitet, so dass der erste Filter 18 umgangen wird.

Vorliegend mündet der zweite Strömungspfad 13 vor dem zweiten Filter 20 in den gemeinsamen Abschnitt 19. Damit wird während der Dekontaminationsphase der erste Filter 18 umgangen und die Luft bzw. das Dekontaminationsmittel ausschließlich über den zweiten Filter 20 geleitet.

Mit anderen Worten, während der Dekontaminationsphase wird die Luft bzw. das Dekontaminationsmittel mittels des zweiten Strömungspfades 13 um den ersten Filter 18 umgeleitet. Das in Figur 6 dargestellte und während der Dekontaminationsphase geschlossene erste Ventil 23 verhindert, dass die Luft bzw. das Dekontaminationsmittel über den ersten Filter 18 strömen kann. Mit anderen Worten, das geschlossene erste Ventil 23 entkoppelt während des Dekontaminationsprozesses den ersten Filter 18 fluidisch von dem Arbeitsraum 16.

Das Dekontaminationsmittel bzw. die Luft aus dem Arbeitsraum 16 wird während des Dekontaminationsprozesses ausschließlich über den zweiten Filter 20 geleitet. Das Dekontaminationsmittel kommt daher nicht in Kontakt mit dem ersten Filter 18 bzw. dessen Beladung.

## Patentansprüche

1. Pharmazeutische Anlage (12), insbesondere ein Reinraum (14), umfassend:
- einen gegenüber seiner Umgebung abgeschlossenen Arbeitsraum (16),
- ein Filtersystem (10) mit einem ersten Strömungspfad (11) und einem ersten Filter (18), wobei der erster Filter (18) in dem ersten Strömungspfad (11) angeordnet ist und ausgebildet ist, um Luft, die aus dem Arbeitsraum (16) über den ersten Strömungspfad (11) geleitet wird, zu filtern, **dadurch gekennzeichnet, dass** das Filtersystem (10) einen zweiten Strömungspfad (13) und eine Umschalteinrichtung (22) umfasst, wobei die Umschalteinrichtung ausgebildet ist, um während einer Betriebsphase der pharmazeutischen Anlage (12), Luft aus dem Arbeitsraum (16) über den ersten Strömungspfad (11) und somit über den ersten Filter (18) zu leiten und während einer Dekontaminationsphase der pharmazeutischen Anlage (12) die Luft aus dem Arbeitsraum (16) ausschließlich über den zweiten Strömungspfad (13) zu leiten, so dass der erste Filter (18) während der Dekontaminationsphase, in der ein, insbesondere gasförmiges, Dekontaminationsmittel in den Arbeitsraum (16) eingespeist wird, nicht mit dem Dekontaminationsmittel, insbesondere mit Luft aus dem Arbeitsraum (16), beaufschlagt wird, wobei Luft, Umluft ist, die im Arbeitsraum (16) rezirkuliert wird, wobei das Filtersystem (10) einen zweiten Filter (20) umfasst, der in dem zweiten Strömungspfad (13) angeordnet ist und ausgebildet ist, um Luft aus dem Arbeitsraum (16), die über den zweiten Strömungspfad (13) geleitet wird, zu filtern, wobei der erste Filter (18) und der zweite Filter (20) fluidisch in Reihe zu einander angeordnet sind, wobei der zweite Filter (20) dem ersten Filter (18) nachgeschaltet ist, insbesondere wobei der zweite Filter (20) ein Polizeifilter (21) ist, wobei der zweite Strömungspfad (13) die Luft aus dem Arbeitsraum (16) parallel um den ersten Filter (18) und durch den zweiten Filter (20) leitet, wobei der erste Strömungspfad (11) und der zweite Strömungspfad (13) einen ersten gemeinsamen Abschnitt (17) und einen zweiten gemeinsamen Abschnitt (19) aufweisen, wobei der zweite Filter (20) innerhalb des gemeinsamen zweiten Abschnitts (19) angeordnet ist.

2. Pharmazeutische Anlage (12) nach Anspruch 1, **dadurch gekennzeichnet, dass** die Dekontaminationsphase eine Bio-Dekontaminationsphase ist, in der, insbesondere gasförmiges, Wasserstoffperoxid, in den Arbeitsraum (16) eingeleitet wird.

3. Pharmazeutische Anlage (12) nach einem der voranstehenden Ansprüche, **dadurch gekennzeichnet, dass** die Umschalteinrichtung (22) mindestens ein Ventil (24, 23, 25), insbesondere zwei Ventile (24, 23, 25), umfasst, wobei das Ventil (24, 23, 25) ausgebildet ist, insbesondere die beiden Ventile (24, 23, 25) ausgebildet sind, um den ersten Strömungspfad (11) und/oder den zweiten Strömungspfad (13) fluidisch mit dem Arbeitsraum (16), zu koppeln und/oder zu entkoppeln.

4. Pharmazeutische Anlage (12) nach einem der voranstehenden Ansprüche, **dadurch gekennzeichnet, dass** der erste Filter (18) und/oder der zweite Filter (20) innerhalb der pharmazeutischen Anlage (12), insbesondere innerhalb des Arbeitsraumes (16), angeordnet ist.

5. Pharmazeutische Anlage (12) nach einem der Ansprüche 1 bis 3, **dadurch gekennzeichnet, dass** der erste Filter (18) und/oder der zweite Filter (20) außerhalb des Arbeitsraumes (16), insbesondere außerhalb der pharmazeutischen Anlage (12), angeordnet ist.

6. Pharmazeutische Anlage (12) nach einem der voranstehenden Ansprüche, **dadurch gekennzeichnet, dass** die Umschalteinrichtung ausgebildet ist, um während der Betriebsphase der pharmazeutischen Anlage (12) Luft aus dem Arbeitsraum (16), über den ersten Filter (18) und den zweiten Filter (20) zu leiten.

7. Verfahren zum Betreiben einer pharmazeutischen Anlage (12) mit einem gegenüber seiner Umgebung abgeschlossenem Arbeitsraum (16), wobei die pharmazeutische Anlage (12) ein Filtersystem (10) umfasst mit einem ersten Strömungspfad (11) und einem ersten Filter (18), wobei der erster Filter (18) in dem ersten Strömungspfad (11) angeordnet ist und ausgebildet ist, um Luft, die aus dem Arbeitsraum (16) über den ersten Strömungspfad (11) geleitet wird, zu filtern, wobei das Filtersystem (10) einen zweiten Strömungspfad (13) und eine Umschalteinrichtung (22) umfasst, wobei mittels der Umschalteinrichtung die Strömung der Luft aus dem Arbeitsraum (16) zwischen dem ersten Strömungspfad (11) und dem zweiten Strömungspfad (13) schaltbar ist, wobei das Verfahren umfasst:
Leiten von Luft aus dem Arbeitsraum (16) über den ersten Strömungspfad (11) in einer Betriebsphase der pharmazeutischen Anlage (12) und Leiten von Luft aus dem Arbeitsraum (16) ausschließlich über den zweiten Strömungspfad (13) während einer Dekontaminationsphase des Arbeitsraumes (16), insbesondere wobei während der Dekontaminationsphase ein, insbesondere gasförmiges,
Dekontaminationsmittel, insbesondere Wasserstoffperoxid, in den Arbeitsraum (16) eingeleitet wird, wobei Luft, Umluft ist, die im Arbeitsraum (16) rezirkuliert wird, wobei zum Betreiben der pharmazeutischen Anlage (12), eine pharmazeutische Anlage (12) nach einem der Ansprüche 1 bis 6 benutzt wird.

8. Verfahren nach Anspruch 7, **dadurch gekennzeichnet, dass** während der Betriebsphase der pharmazeutischen Anlage (12) Luft aus dem Arbeitsraum (16) zusätzlich über den zweiten Strömungspfad (13) geleitet wird.

## Claims

1. Pharmaceutical facility (12), in particular a cleanroom (14), comprising:
- a working space (16) closed off from its surroundings,
- a filter system (10) with a first flow path (11) and a first filter (18), wherein the first filter (18) is arranged in the first flow path (11) and is designed to filter air which is conducted out of the working space (16) via the first flow path (11), **characterized in that** the filter system (10) comprises a second flow path (13) and a switching device (22), wherein the switching device is designed to conduct air out of the working space (16) via the first flow path (11) and thus via the first filter (18) during an operating phase of the pharmaceutical facility (12) and to conduct the air out of the working space (16) exclusively via the second flow path (13) during a decontamination phase of the pharmaceutical facility (12), such that the first filter (18) is not exposed to the decontamination agent, in particular to air from the working space (16), during the decontamination phase in which an in particular gaseous decontamination agent is supplied to the working space (16), wherein air is circulating air which is recirculated in the working space (16), wherein the filter system (10) comprises a second filter (20) which is arranged in the second flow path (13) and is designed to filter air from the working space (16) which is conducted via the second flow path (13), wherein the first filter (18) and the second filter (20) are arranged fluidically in series with one another, wherein the second filter (20) is connected downstream of the first filter (18), in particular wherein the second filter (20) is a safety filter (21), wherein the second flow path (13) conducts the air out of the working space (16) in parallel around the first filter (18) and through the second filter (20), wherein the first flow path (11) and the second flow path (13) have a first common portion (17) and a second common portion (19), wherein the second filter (20) is arranged within the common second portion (19).

2. Pharmaceutical facility (12) according to claim 1, **characterized in that** the decontamination phase is an eco-decontamination phase in which in particular gaseous hydrogen peroxide is introduced into the working space (16).

3. Pharmaceutical facility (12) according to either of the preceding claims, **characterized in that** the switching device (22) comprises at least one valve (24, 23, 25), in particular two valves (24, 23, 25), wherein the valve (24, 23, 25) is designed, in particular the two valves (24, 23, 25) are designed, to fluidically couple and/or decouple the first flow path (11) and/or the second flow path (13) to/from the working space (16).

4. Pharmaceutical facility (12) according to any of the preceding claims, **characterized in that** the first filter (18) and/or the second filter (20) is arranged within the pharmaceutical facility (12), in particular within the working space (16).

5. Pharmaceutical facility (12) according to any of claims 1 to 3, **characterized in that** the first filter (18) and/or the second filter (20) is arranged outside the working space (16), in particular outside the pharmaceutical facility (12).

6. Pharmaceutical facility (12) according to any of the preceding claims, **characterized in that** the switching device is designed to conduct air out of the working space (16) via the first filter (18) and the second filter (20) during the operating phase of the pharmaceutical facility (12).

7. Method for operating a pharmaceutical facility (12) with a working space (16) closed off from its surroundings, wherein the pharmaceutical facility (12) comprises a filter system (10) with a first flow path (11) and a first filter (18), wherein the first filter (18) is arranged in the first flow path (11) and is designed to filter air which is conducted out of the working space (16) via the first flow path (11), wherein the filter system (10) comprises a second flow path (13) and a switching device (22), wherein the flow of the air from the working space (16) can be switched between the first flow path (11) and the second flow path (13) by means of the switching device, wherein the method comprises:
conducting air out of the working space (16) via the first flow path (11) in an operating phase of the pharmaceutical facility (12) and conducting air out of the working space (16) exclusively via the second flow path (13) during a decontamination phase of the working space (16), in particular wherein an in particular gaseous decontamination agent, in particular hydrogen peroxide, is introduced into the working space (16) during the decontamination phase, wherein air is circulating air which is recirculated in the working space (16), wherein in order to operate the pharmaceutical facility (12), a pharmaceutical facility (12) according to any of claims 1 to 6 is used.

8. Method according to claim 7, **characterized in that** air is conducted out of the working space (16) additionally via the second flow path (13) during the operating phase of the pharmaceutical facility (12).

## Revendications

1. Installation pharmaceutique (12), en particulier une salle blanche (14), comprenant :
- un espace de travail (16) isolé de son environnement,
- un système de filtration (10) comportant un premier circuit d'écoulement (11) et un premier filtre (18), dans lequel le premier filtre (18) est disposé dans le premier circuit d'écoulement (11) et est conçu pour filtrer l'air canalisé depuis l'espace de travail (16) par l'intermédiaire du premier circuit d'écoulement (11), **caractérisée en ce que** le système de filtration (10) comprend un second circuit d'écoulement (13) et un dispositif de commutation (22), dans laquelle le dispositif de commutation est conçu pour, pendant une phase de fonctionnement de l'installation pharmaceutique (12), canaliser l'air provenant de l'espace de travail (16) par l'intermédiaire du premier circuit d'écoulement (11) et donc par l'intermédiaire du premier filtre (18), et pour, pendant une phase de décontamination de l'installation pharmaceutique (12), canaliser l'air provenant de l'espace de travail (16) exclusivement par l'intermédiaire du second circuit d'écoulement (13), en sorte que le premier filtre (18), pendant la phase de décontamination au cours de laquelle un agent de décontamination, en particulier gazeux, est injecté dans l'espace de travail (16), ne soit pas exposé à l'agent de décontamination, en particulier à l'air provenant de l'espace de travail (16), dans laquelle l'air, est de l'air de recirculation qui est recirculé dans l'espace de travail (16), dans laquelle le système de filtration (10) comprend un second filtre (20) qui est disposé dans le second circuit d'écoulement (13) et qui est conçu pour filtrer l'air provenant de l'espace de travail (16), qui est canalisé par le second circuit d'écoulement (13), dans laquelle le premier filtre (18) et le second filtre (20) sont disposés en série, fluidiquement l'un par rapport à l'autre, dans lequel le second filtre (20) est monté en aval du premier filtre (18), en particulier dans laquelle le second filtre (20) étant un filtre de sécurité (21), dans laquelle le second circuit d'écoulement (13) canalise l'air provenant de l'espace de travail (16) parallèlement en dérivation du premier filtre (18) et à travers le second filtre (20), dans laquelle le premier circuit d'écoulement (11) et le second circuit d'écoulement (13) présentent une première section commune (17) et une seconde section commune (19), dans laquelle le second filtre (20) est disposé à l'intérieur de la seconde section commune (19).

2. Installation pharmaceutique (12) selon la revendication 1,
**caractérisée en ce que** la phase de décontamination est une phase de biodécontamination au cours de laquelle du peroxyde d'hydrogène, en particulier sous forme gazeuse, est introduit dans l'espace de travail (16).

3. Installation pharmaceutique (12) selon l'une des revendications précédentes, **caractérisée en ce que** le dispositif de commutation (22) comprend au moins une vanne (24, 23, 25), en particulier deux vannes (24, 23, 25), dans laquelle la vanne (24, 23, 25) est conçue, en particulier les deux vannes (24, 23, 25) sont conçues pour accoupler et/ou désaccoupler fluidiquement le premier circuit d'écoulement (11) et/ou le second circuit d'écoulement (13) à/de l'espace de travail (16).

4. Installation pharmaceutique (12) selon l'une des revendications précédentes, **caractérisée en ce que** le premier filtre (18) et/ou le second filtre (20) sont disposés à l'intérieur de l'installation pharmaceutique (12), en particulier à l'intérieur de l'espace de travail (16).

5. Installation pharmaceutique (12) selon l'une des revendications 1 à 3, **caractérisée en ce que** le premier filtre (18) et/ou le second filtre (20) sont disposés à l'extérieur de l'espace de travail (16), en particulier à l'extérieur de l'installation pharmaceutique (12).

6. Installation pharmaceutique (12) selon l'une des revendications précédentes, **caractérisée en ce que** le dispositif de commutation est conçu**,** pendant la phase de fonctionnement de l'installation pharmaceutique (12), pour canaliser l'air provenant de l'espace de travail (16), par l'intermédiaire du premier filtre (18) et du second filtre (20).

7. Procédé permettant de faire fonctionner une installation pharmaceutique (12) comportant un espace de travail (16) isolé de son environnement, dans lequel l'installation pharmaceutique (12) comprend un système de filtration (10) comportant un premier circuit d'écoulement (11) et un premier filtre (18), dans lequel le premier filtre (18) est disposé dans le premier circuit d'écoulement (11) et conçu pour filtrer l'air canalisé depuis l'espace de travail (16) par l'intermédiaire du premier circuit d'écoulement (11), dans lequel le système de filtration (10) comprend un second circuit d'écoulement (13) et un dispositif de commutation (22), dans lequel le dispositif de commutation permet de commuter l'écoulement de l'air provenant de l'espace de travail (16) entre le premier circuit d'écoulement (11) et le second circuit d'écoulement (13), dans lequel le procédé comprend :
la canalisation de l'air provenant de l'espace de travail (16) par l'intermédiaire du premier circuit d'écoulement (11) pendant une phase de fonctionnement de l'installation pharmaceutique (12) et la canalisation de l'air provenant de l'espace de travail (16) exclusivement par l'intermédiaire du second circuit d'écoulement (13) pendant une phase de décontamination de l'espace de travail (16), en particulier dans lequel, pendant la phase de décontamination, un agent de décontamination, en particulier gazeux, en particulier du peroxyde d'hydrogène, est introduit dans l'espace de travail (16), dans lequel l'air est de l'air de recirculation qui est recirculé dans l'espace de travail (16), dans lequel, pour faire fonctionner l'installation pharmaceutique (12), une installation pharmaceutique (12) selon l'une des revendications 1 à 6 est utilisée.

8. Procédé selon la revendication 7, **caractérisé en ce que,** pendant la phase de fonctionnement de l'installation pharmaceutique (12), l'air provenant de l'espace de travail (16) est en outre canalisé par l'intermédiaire du second circuit d'écoulement (13).
